# EUROPEAN PATENT SPECIFICATION

(11) **EP 4 051 186 B1**
(45) Date of publication and mention of the grant of the patent: **03.12.2025**
(21) Application number: 20800736.9
(22) Date of filing: 21.10.2020
(51) Int. Cl.: A61F 2/42

(54) **PROSTHESIS FOR MOVABLY COUPLING OS METACARPALE I OF A HAND TO A SECOND BONE OF THE HAND AND A KIT COMPRISING A PROSTHESIS AND A FASTENING MEANS**
PROTHESE ZUM BEWEGLICHEN KOPPELN DES OS METACARPALE I EINER HAND MIT EINEM ZWEITEN KNOCHEN DER HAND UND EIN BESTEHEND AUS PROTHESE UND EINEM BEFESTIGUNGSMITTEL
PROTHÈSE POUR LE COUPLAGE MOBILE DE L'OS MÉTACARPIEN I D'UNE MAIN À UN SECOND OS DE LA MAIN ET UN KIT COMPRENANT UNE PROTHÈSE ET UN MOYEN DE FIXATION

(30) Priority: 28.10.2019 NL 2024112
(43) Date of publication of application: 07.09.2022
(73) Proprietor: De Jong, Tjeerd René, 8033 PA Zwolle (NL)
(72) Inventor: De Jong, Tjeerd René, 8033 PA Zwolle (NL)
(74) Representative: 't Jong, Bastiaan Jacob
(86) International application number: PCT/NL2020/050651
(87) International publication number: WO 2021/086176

(56) References cited:
- US-A1- 2013 197 655
- US-A1- 2016 302 935
- US-A1- 2018 168 814
- US-A1- 2019 167 437

## Description

The invention relates to a prosthesis for movably coupling os metacarpale I of a hand to a second bone of the hand, which prosthesis comprises a first and a second element, wherein the first element comprises a first attachment member, which first attachment member is configured for attachment to os metacarpale I, and the second element comprises a second attachment member, which second attachment member is configured for attachment to the second bone of the hand, which first and second element are movable relative to each other and wherein the second bone of the hand is os 15 metacarpale II of the hand. The closest prior art is document US 2016/302935 A1, which defines the preamble of claim 1.

Thumb base prostheses are generally known for the treatment of osteoarthritis of the thumb basal joint, i.e. the first carpometacarpal joint (CMC1). In known thumb base prostheses the damaged CMC1 joint is replaced with a ball-and-socket joint. A cup is here operatively placed in os trapezium, and a shaft with neck and head in os metacarpale I. This guarantees that the thumb has good mobility and also keeps its length.

Such prostheses however do not produce the desired result if they are applied when the underlying scaphotrapeziotrapezoid (STT) joint also exhibits arthritic changes. This is because, if the CMC1 is replaced, the osteoarthritis in the STT joint remains and the patient can continue to have adverse symptoms.

Recent literature shows that a trapeziectomy is an effective treatment for osteoarthritis of the CMC1 joint. The os trapezium is completely removed herein. Joint replacement surgery is however no longer an option for those who have developed symptoms due to the collapse of os metacarpale I, since there no longer is an os trapezium. It is no longer possible to place a cup.

It is now an object of the invention to provide a prosthesis wherein at least one of the above stated drawbacks is reduced or even obviated.

This object is achieved according to the invention, which is defined in claim 1.

By attaching the second element of the prosthesis to os metacarpale II (MC2) by means of the second attachment member the mobility of the thumb can remain guaranteed after a trapeziectomy, without the usual shortening of the thumb occurring. In attachment to os metacarpale II the axial forces which the flexor tendons and extensor tendons of the thumb normally transmit via the CMC1 and the STT joint to the radius, are diverted via the capitate to the proximal carpal row and to the radius.

Particularly advantageous is arranging the second attachment member from the radial side of os metacarpale II. This side of os metacarpale II can be made accessible during surgery by means of a simple dissection. The joint surface between os metacarpale II and the trapezoid bone can remain untouched here. Attachment to the radial side of os metacarpale II further makes it possible to remove the os trapezium, whereby no further strain on affected joint surfaces occurs. Patients who also suffer symptoms of arthritis on the underside of the os trapezium, also referred to as pan-arthritis of the os trapezium, are hereby no longer dependent on the os trapezium being preserved for placement of a joint cup.

The prosthesis according to the invention also provides a heretofore unavailable stable salvage option for patients having symptoms of collapse after a trapeziectomy. The suspensionplasties which are currently implemented to prevent this secondary collapse often have disappointing results. The same is true for arthrodesis between os metacarpale I and os metacarpale II, wherein a high percentage of non-unions is observed and wherein movement in the thumb basal joint is no longer possible. The arrangement on os metacarpale II in order to form a replacement base for a thumb basal joint ensures that a good mobility of the thumb can be restored in said problem cases.

The first attachment member can be arranged on or in os metacarpale I by means of per se known methods.

In accordance with medical nomenclature, the radial side of os metacarpale II is the side located on the thumb side, so the lateral side of the hand where the radius, also referred to as the spoke-bone, runs to the wrist.

Another embodiment of a prosthesis according to the invention is a prosthesis wherein the second attachment member comprises a plate, which plate corresponds on a first side with the curved shape of the radial outer side of os metacarpale II of the hand.

Attaching a plate to the outer side of os metacarpale II can provide a base for the above stated diversion route. This plate, which is preferably preformed, follows the curved shape of os metacarpale II and is anchored thereto with angular stability.

Another embodiment of a prosthesis according to the invention is a prosthesis wherein the plate comprises at least one fastening hole for receiving a fastening means such as for instance a screw, pin or peg, wherein the at least one fastening hole is continuous and extends to the first side.

Providing the plate with a fastening hole, preferably at least two fastening holes, for use with fastening means such as for instance screws enables the plate to be pulled very firmly against the radial side of MC2. The fastening holes are preferably distributed evenly over the plate, so that the plate can be anchored at least distally and proximally and, if necessary, also therebetween.

The continuous fastening holes extend to the first side, whereby the first side can be pulled tightly against the radial side of os metacarpale II. A strong connection can thus be obtained between the plate of the second attachment member and os metacarpale II.

Per se known screws can be used for this purpose, such as for instance bicortical, unicortical or unicortical far-cortex-abutting screws, optionally in a locking embodiment and optionally coated at least partially with hydroxyapatite. The screws can also be embodied as biocomposite screws of for instance hydroxyapatite and tricalcium phosphate for improved osseointegration.

On the side lying opposite the first side the fastening holes can be shaped for guiding and/or countersinking of the fastening means.

Another prosthesis according to the invention is an embodiment wherein at least one of the at least one fastening hole is provided with first locking means, such as for instance internal screw thread or a relief structure, for the purpose of anchoring a fastening means to the plate with angular stability relative to the plate.

In order to prevent the plate from being able to tilt relative to the fastening means, wherein the angle between the plate and the fastening means changes, it is advantageous to provide fastening holes with locking means. The locking means enable the angle between the fastening means and the plate to be fixed, whereby a connection with angular stability can thus be achieved between the plate and the fastening means.

The locking means can for instance comprise internal screw thread, wherein the internal screw thread corresponds with screw thread arranged on the fastening means. The internal screw thread can for instance be self-locking. A relief structure can alternatively or additionally also provide or enhance the desired angular stability, wherein the relief structure provides for increased friction during attachment, or even deformation of the fastening means for the purpose of obtaining the locking effect.

In another embodiment of a prosthesis according to the invention the plate comprises at least one fastening pin, which at least one fastening pin extends from the first side and is configured to attach the plate to os metacarpale II.

In order to simplify the placing of the prosthesis a fastening pin can be provided on the first side of the plate, which side abuts os metacarpale II during use. The fastening pin can be coated at least partially with hydroxyapatite for the purpose of obtaining a good osseointegration of the fastening pin in the bone tissue.

The fastening pin can be placed at right angles relative to the first side, but it is also possible to choose a different angle, for instance for the purpose of simple placement, wherein the fastening pin serves as a guide.

The plate is also attached to os metacarpale II by means of the fastening pin. By making a corresponding drilled hole in os metacarpale II, for instance using a drill jig or drill guide, the fastening pin can also attach or anchor the plate to os metacarpale II. It is known how a form-fitting connection between the fastening pin and bone tissue can be obtained.

In a preferred embodiment of a prosthesis according to the invention the second attachment member comprises an intramedullary rod, which intramedullary rod is elongate and is configured for intramedullary insertion, via the joint surface of os metacarpale II and the trapezium, into the medullary cavity of os metacarpale II.

Arranging the second element in the medullary cavity of os metacarpale II from the radial side of os metacarpale II by means of an intramedullary rod of the second attachment member makes it possible to work with a smaller dissection. When performing a trapeziectomy, the joint surface of os metacarpale II and os trapezium becomes easily accessible. The medullary cavity can be made accessible and made to fit by making use of a flexible reamer, or reaming drill, as is usual in known drilled techniques.

In order to enable sufficient insertion depth of the intramedullary rod a medullary space is needed. This space of the medullary cavity can optionally be made to fit by means of a flexible cannulated reamer.

Nor does the intrinsic musculature, such as the first dorsal interosseus, have to be moved off os metacarpale II, as is the case when another embodiment, wherein the second attachment member comprises a plate, is attached. Owing to the largely intraosseous placement, the chance that the second attachment member can be felt through the skin is also smaller.

Another embodiment according to the invention is a prosthesis wherein the intramedullary rod is curved, wherein the curvature corresponds with the curvature of the medullary cavity of os metacarpale II.

Giving the intramedullary rod a curved shape which corresponds with the curvature of the inner wall of the medullary cavity of os metacarpale II prevents rotation of the intramedullary rod relative to os metacarpale II. The curved or bent shape is preferably preformed.

In another embodiment of a prosthesis according to the invention the intramedullary rod is provided at least partially with a hydroxyapatite coating.

In order to ensure an optimal connection between the attachment member and the bone material of the medullary cavity the intramedullary rod can be provided with hydroxyapatite. This promotes osseointegration.

In a preferred embodiment of a prosthesis according to the invention the intramedullary rod comprises at least fixing holes for the purpose of anchoring the intramedullary rod in the medullary cavity of os metacarpale II with angular stability, wherein the at least two fixing holes are arranged at right angles relative to the longitudinal axis of the intramedullary rod and wherein the respective axes of the fixing holes are arranged at an angle greater than 5° relative to each other, preferably at an angle of between 45° and 135°, preferably at an angle of 90°.

The intramedullary rod can be anchored to os metacarpale II with angular stability by means of fixing holes provided in the intramedullary rod. For this purpose the cortex of os metacarpale II is drilled through in minimally invasive manner, for instance with a device or jig configured for this purpose. Fastening means such as for instance screws can be arranged through these drilled holes. The fixing holes can for this purpose be provided with internal screw thread.

In order to obtain a fixation with angular stability of the second element relative to os metacarpale II it is desirable to secure the intramedullary rod with at least two fastening means arranged at an angle relative to each other. In order to obtain the desired angular stability the angle must be at least 5°. Such a small angle can be desirable for minimally invasive drilling or avoiding vital structures such as nerves or bloodstreams. The angle preferably lies between 45° and 135°, whereby a better fixation relative to two axes is achieved. The angle is preferably about 90°. In the case of such an angle a highly effective fixation against rotation is even obtained with only two fastening means, such as screws or bolts.

Another embodiment according to the invention is a prosthesis wherein the second element also comprises a third attachment member configured for attachment to os trapezoideum of the hand.

When a third attachment member to be attached to os trapezoideum is also provided in proximal direction relative to the second attachment member, when attached to os metacarpale II, the forces can optionally also be diverted proximally partially via os trapezoideum. This third attachment member can be made suitable for attachment to os trapezoideum in similar manner to the second attachment member.

Another embodiment according to the invention is a prosthesis wherein the first element is coupled to the second element by a ball-and-socket joint.

Since the thumb basal joint is a saddle joint which allows a lot of movement, such as flexion, extension, abduction, adduction and opposition, it is desirable to apply a ball-and-socket joint for the purpose of coupling the first and second element which are movable relative to each other. A ball-and-socket joint comprises here an at least partially cup-shaped cavity and a corresponding, at least partially spherical ball which is arranged rotatably in the cavity.

The prosthesis can also be embodied as salvage option in the case of failure of the proximal component of an existing prosthesis. For instance in the case of malposition of the cup, when it has come loose, or when it has become unusable due to wear. A cup, for instance arranged in os trapezium, can hereby be replaced in simple manner by the second element of the prosthesis according to the invention, wherein a joint part matching the joint replacement already arranged in os metacarpale I is used on the second element, such as for instance a fitting cup. The first element, attached to os metacarpale I, is then in fact reused from the existing prosthesis.

In another embodiment of a prosthesis according to the invention the ball-and-socket joint comprises a first part, which first part comprises an at least partially cup-shaped cavity and which first part is arranged on the first element, and wherein the ball-and-socket joint comprises a second part, which second part comprises an at least partially spherical ball, which second part is arranged on the second element and wherein the spherical part of the ball is arranged rotatably in the cavity.

In known prostheses for replacement of the thumb basal joint a cup is arranged in the os trapezium. A pin with ball head is then attached to the proximal outer end of os metacarpale I. In existing prostheses malposition or loosening of the cup commonly occurs in the course of time, this resulting in luxation and adverts symptoms. Although this known orientation can also be realized with the prosthesis according to the invention, provisions are also made for this attachment to be reversed. The cup is here attached to the first element and the ball to the second element.

With the mechanical fixation of the first attachment member, which is for instance wedge-shaped and is arranged in the proximal outer end of os metacarpale I, and attachment of the first part of the ball-and-socket joint, which part comprises the cup, to the first element, malposition is no longer a problem. On the contrary, the axial load occurring on the cup strengthens the attachment of the cup.

The invention also relates to a combination of a prosthesis according to the invention and at least one fastening means, such as an at least partially hydroxyapatite-coated peg, wherein the at least one fastening means is elongate and is provided on or close to a first outer end with second locking means for the purpose of anchoring the at least one fastening means with angular stability in at least one of the at least one fastening hole provided with first locking means, and is configured at a second outer end for attachment of the plate to os metacarpale II by means of a form-fitting connection.

For the purpose of attaching the plate of the second attachment member to os metacarpale II provisions are made for a peg to be provided with second locking means. These locking means can correspond with first locking means optionally provided in the fastening holes of the second attachment member. The locking means can for instance be screw thread or a relief structure.

A peg can be anchored in the plate with angular stability by the locking means. A suitable peg can hereby be chosen for arranging of the prosthesis, after which this peg is anchored in the plate. The peg can then serve as a guide in the placing of the prosthesis, and provide for attachment of the second attachment member of the prosthesis.

These and other features of the invention are further elucidated with reference to the accompanying drawings.
Figure 1 shows a perspective view of a first embodiment of the prosthesis according to the invention.
Figure 2 shows a perspective view of a first embodiment of the prosthesis according to the invention, placed in a hand.
Figure 3 shows a perspective view of a second embodiment of the prosthesis according to the invention.
Figure 4 shows a perspective view of a third embodiment of the prosthesis according to the invention.
Figure 5 shows a perspective view of a third embodiment of the prosthesis according to the invention, placed in a hand.

In the figure description os metacarpale I and os metacarpale II are designated as respectively MC1 and MC2.

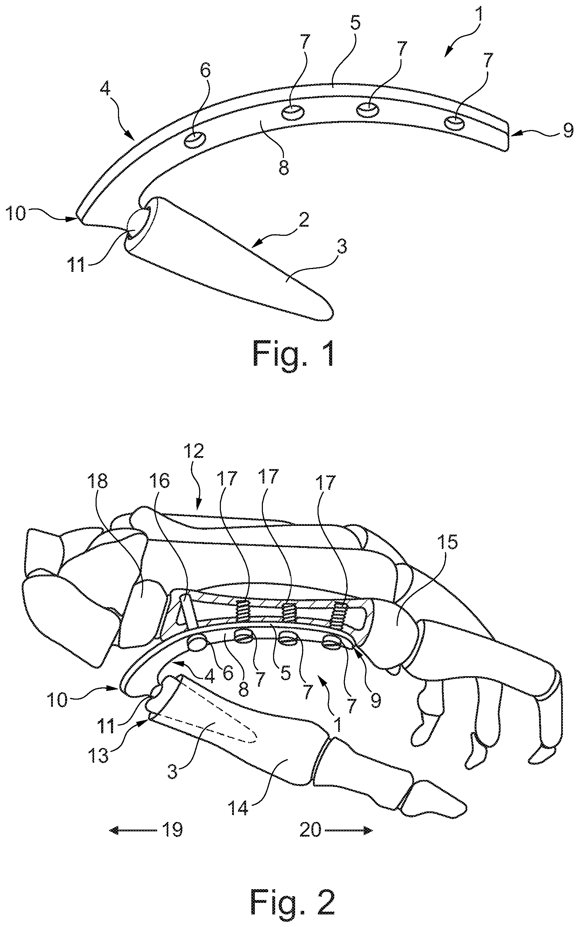

Figure 1 shows a first embodiment of a prosthesis 1 according to the invention. Prosthesis 1 has a first element 2 with a first attachment member 3. Second element 4 has a second attachment member 5 in the form of an elongate plate. Continuous fastening holes 6, 7 are arranged evenly in the plate for the purpose of receiving fastening means. The curvature of the plate is clearly visible, wherein the curvature of the plate corresponds on the mounting side, also referred to as first side, to the slightly concave form of the radial side of MC2. The curvature of the mounting side of the plate thus corresponds at least to the curvature of the radial side of MC2 in the coronal plane. When second attachment member 5 is attached to MC2, the mounting side or first side will have an ulnar orientation. The side 8 of second attachment member 5 lying opposite thereto has a radial orientation.

The distal end 9 of the second element ends close to a fastening hole 7. Arranged close to proximal end 10 of the second element is a partially spherical head 11 which forms a ball-and-socket joint with the cup arranged in second element 2.

Figure 2 shows a first embodiment of the prosthesis 1 according to the invention, placed in a hand 12. First attachment member 3 is arranged in the proximal end 13 of MC1 14. Os trapezium has already been removed. The second attachment member 5 is attached to MC2 15 by means of fastening means 16, 17 arranged in fastening holes 6, 7. It can be clearly seen in the partly cut-away MC2 15 that the bicortical screws 17 connect the second attachment member 5 fixedly to the radial side of MC2 15. A pin 16 is arranged in the most proximally situated fastening hole 6. It is clearly visible that prosthesis 1 provides for diversion of the forces via MC2 15 and os trapezoideum 18 in proximal direction 19, designated with arrow 19. The distal direction 20 is designated with arrow 20.

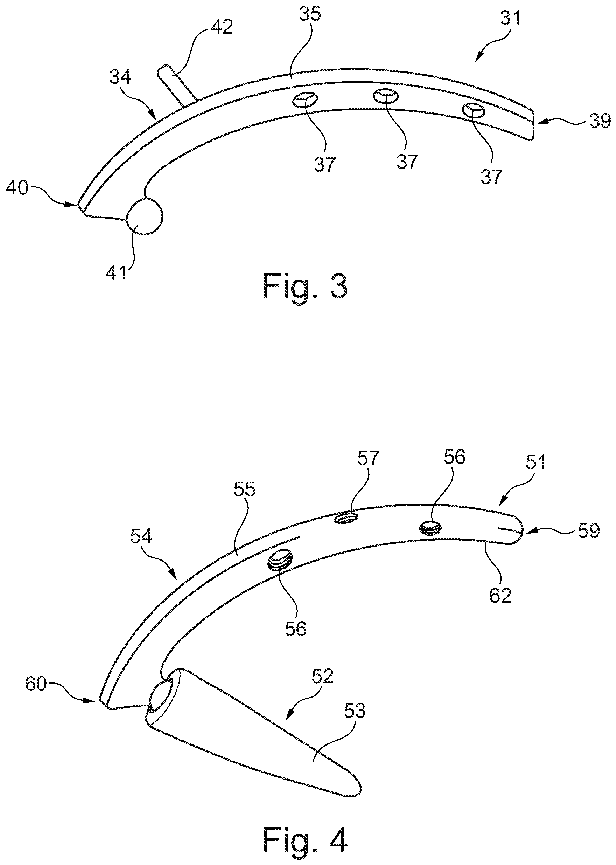

Figure 3 shows a second embodiment of the prosthesis 31 according to the invention. The first element of the prosthesis is not shown in this figure. The second element 34 has a second attachment member 35, in which continuous fastening holes 37 are arranged evenly for the purpose of receiving fastening means. The continuous fastening holes 37 are arranged at the distal end 39 of the second attachment member 35. Also arranged toward the proximal end 40, on which the ball head 41 of the ball-and-socket joint is also arranged, is a fastening pin 42. This fastening pin 42 forms a whole with the second element 34.

Figure 4 shows a third embodiment of the prosthesis 51 according to the invention. Prosthesis 51 has a first element 52 with a first attachment member 53. Second element 54 has a second attachment member 55 in the form of an intramedullary rod 62. Arranged in the intramedullary rod 62 are optionally continuous fixing holes 56, 57 for receiving fastening means. Fixing holes 56, 57 are arranged at an angle relative to each other. In the shown embodiment the fixing hole 56 close to distal end 59 of second element 54 and the fixing hole 57 lying adjacently thereof in the direction of proximal end 60 form an angle of about 90° with each other in more or less parallel planes, as seen from the longitudinal axis of the elongate intramedullary rod 62.

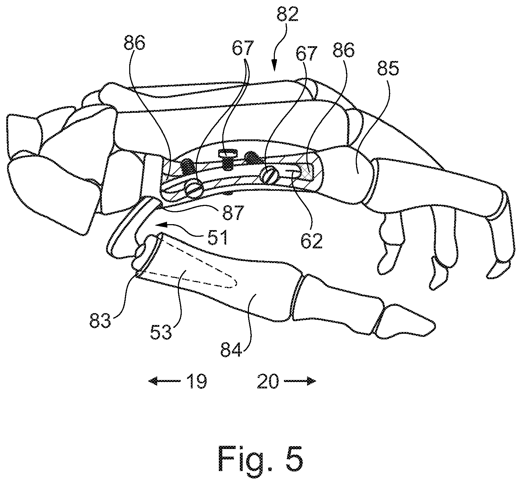

Figure 5 shows a third embodiment of the prosthesis 51 according to the invention, placed in a hand 82. The first attachment member 53 is arranged in the proximal end 83 of MC1 84. Os trapezium has already been removed. The second attachment member 55 in the form of intramedullary rod 62 is arranged in the medullary cavity 86 of MC2 85 through insertion via the radial side of MC2 85, through the joint surface 87 situated between MC2 85 and the already removed os trapezium. In the partially cut-away MC2 85 it is clearly visible that the intramedullary rod 62 follows the internal curvature of medullary cavity 86, whereby rotation is already prevented. Fixing screws 67, which are arranged at an angle relative to each other, fix the intramedullary rod 62 relative to MC2 85 with angular stability. Alternatively, bolts and blind fixing holes can also be used for the fixing. The proximal 19 and distal 20 directions are designated with arrows 19, 20.

## Claims

1. Prosthesis (1; 31; 51) for movably coupling os metacarpale I (14; 84) of a hand (12; 82) to a second bone of the hand (12; 82), which prosthesis (1; 31; 51) comprises a first (2; 52) and a second element (4; 34; 54), wherein the first element (2; 52) comprises a first attachment member (3; 53), which first attachment member (3; 53) is configured for attachment to os metacarpale I (14; 84), and the second element (4; 34; 54) comprises a second attachment member (5; 35; 55), which second attachment member (5; 35; 55) is configured for attachment to the second bone of the hand (12; 82), which first (2; 52) and second element (4; 34; 54) are movable relative to each other and wherein the second bone of the hand (12; 82) is os metacarpale II (15; 85) of the hand (12; 82), **characterized in that** the second attachment member (5; 35; 55) is configured to be attached to the radial side of os metacarpale II (15; 85).

2. Prosthesis (1; 31) according to claim 1, wherein the second attachment member (4; 34) comprises a plate (5; 35), which plate (5; 35) corresponds on a first side with the curved shape of the radial outer side of os metacarpale II (15) of the hand (12).

3. Prosthesis (1; 31) according to claim 2, wherein the plate (5; 35) comprises at least one fastening hole (6, 7; 37) for receiving a fastening means such as for instance a screw, pin or peg, wherein the at least one fastening hole (6, 7; 37) is continuous and extends to the first side.

4. Prosthesis (1; 31) according to claim 3, wherein at least one of the at least one fastening hole (6, 7; 37) is provided with first locking means, such as an internal screw thread or a relief structure, for the purpose of anchoring a fastening means to the plate (5; 35) with angular stability relative to the plate (5; 35).

5. Prosthesis (1; 31) according to any one of the claims 2-4, wherein the plate (5; 35) comprises at least one fastening pin (16; 42), which at least one fastening pin (16; 42) extends from the first side and is configured to attach the plate (5; 35) to os metacarpale II (15).

6. Prosthesis (51) according to claim 1, wherein the second attachment member (55) comprises an intramedullary rod (62), which intramedullary rod (62) is elongate and is configured for intramedullary insertion, via the joint surface of os metacarpale II (85) and the trapezium, into the medullary cavity (86) of os metacarpale II (85).

7. Prosthesis (51) according to claim 6, wherein the intramedullary rod (62) is curved, wherein the curvature corresponds with the curvature of the medullary cavity (86) of os metacarpale II (85).

8. Prosthesis (51) according to any one of the claims 6 or 7, wherein the intramedullary rod (62) is provided at least partially with a hydroxyapatite coating.

9. Prosthesis (51) according to any one of the claims 6-8, wherein the intramedullary rod (62) comprises at least two fixing holes (56, 57) for the purpose of anchoring the intramedullary rod (62) in the medullary cavity (86) of os metacarpale II (85) with angular stability, wherein the at least two fixing holes (56, 57) are arranged at right angles relative to the longitudinal axis of the intramedullary rod (62) and wherein the respective axes of the fixing holes (56, 57) are arranged at an angle greater than 5° relative to each other, preferably at an angle of between 45° and 135°, preferably at an angle of 90°.

10. Prosthesis (1; 31; 51) according to any one of the foregoing claims, wherein the second element (4; 34; 54) also comprises a third attachment member configured for attachment to os trapezoideum of the hand (12; 82).

11. Prosthesis (1; 31; 51) according to any one of the foregoing claims, wherein the first element (2; 52) is coupled to the second element (4; 34; 54) by a ball-and-socket joint.

12. Prosthesis (1; 31; 51) according to claim 11, wherein the ball-and-socket joint comprises a first part, which first part comprises an at least partially cup-shaped cavity and which first part is arranged on the first element (2; 52), and wherein the ball-and-socket joint comprises a second part, which second part comprises an at least partially spherical ball (11; 41), which second part is arranged on the second element (4; 34; 54) and wherein the spherical part of the ball (11; 41) is arranged rotatably in the cavity.

13. Kit comprising a prosthesis (1; 31) according to any one of the claims 4 or 5 and at least one fastening means (16), such as an at least partially hydroxyapatite-coated peg (16), wherein the at least one fastening means (16) is elongate and is provided on or close to a first outer end with second locking means for the purpose of anchoring the at least one fastening means (16) with angular stability in at least one of the at least one fastening hole (6, 7; 37) provided with first locking means, and is configured at a second outer end for attachment of the plate (5) to os metacarpale II (15) by means of a form-fitting connection.

## Patentansprüche

1. Prothese (1; 31; 51) zum beweglichen Koppeln des Os metacarpale I (14; 84) einer Hand (12; 82) mit einem zweiten Knochen der Hand (12; 82), wobei die Prothese (1; 31; 51) ein erstes (2; 52) und ein zweites Element (4; 34; 54) umfasst, wobei das erste Element (2; 52) ein erstes Befestigungselement (3; 53) umfasst, wobei das erste Befestigungselement (3; 53) zur Befestigung an Os metacarpale I (14; 84) konfiguriert ist, und das zweite Element (4; 34; 54) ein zweites Befestigungselement (5; 35; 55) umfasst, wobei das zweite Befestigungselement (5; 35; 55) zur Befestigung an dem zweiten Knochen der Hand (12; 82) konfiguriert ist, wobei das erste (2; 52) und das zweite Element (4; 34; 54) relativ zueinander bewegbar sind und wobei der zweite Knochen der Hand (12; 82) der Os metacarpale II (15; 85) der Hand (12; 82) ist, **dadurch gekennzeichnet, dass** das zweite Befestigungselement (5; 35; 55) konfiguriert ist, um an der radialen Seite des Os metacarpale II (15; 85) befestigt zu werden.

2. Prothese (1; 31) nach Anspruch 1, wobei das zweite Befestigungselement (4; 34) eine Platte (5; 35) umfasst, wobei die Platte (5; 35) auf einer ersten Seite mit der gekrümmten Form der radialen Außenseite des Os metacarpale II (15) der Hand (12) übereinstimmt.

3. Prothese (1; 31) nach Anspruch 2, wobei die Platte (5; 35) mindestens ein Befestigungsloch (6, 7; 37) zum Aufnehmen eines Befestigungsmittels, wie beispielsweise einer Schraube, eines Stifts oder eines Zapfens, umfasst, wobei das mindestens eine Befestigungsloch (6, 7; 37) durchgehend ist und sich zur ersten Seite erstreckt.

4. Prothese (1; 31) nach Anspruch 3, wobei mindestens eines der mindestens einen Befestigungslöcher (6, 7; 37) mit ersten Verriegelungsmitteln, wie beispielsweise einem Innengewinde oder einer Reliefstruktur, zum Zwecke der Verankerung eines Befestigungsmittels an der Platte (5; 35) mit Winkelstabilität relativ zur Platte (5; 35) versehen ist.

5. Prothese (1; 31) nach einem der Ansprüche 2-4, wobei die Platte (5; 35) mindestens einen Befestigungsstift (16; 42) umfasst, wobei sich der mindestens eine Befestigungsstift (16; 42) von der ersten Seite erstreckt und zum Befestigen der Platte (5; 35) am Os metacarpale II (15) konfiguriert ist.

6. Prothese (51) nach Anspruch 1, wobei das zweite Befestigungselement (55) einen intramedullären Stab (62) umfasst, wobei der intramedulläre Stab (62) länglich ist und zur intramedullären Insertion, über die Gelenkfläche des Os metacarpale II (85) und des Trapeziums, in die Markhöhle (86) des Os metacarpale II (85) konfiguriert ist.

7. Prothese (51) nach Anspruch 6, wobei der intramedulläre Stab (62) gekrümmt ist, wobei die Krümmung mit der Krümmung der Markhöhle (86) des Os metacarpale II (85) übereinstimmt.

8. Prothese (51) nach einem der Ansprüche 6 oder 7, wobei der intramedulläre Stab (62) mindestens teilweise mit einer Hydroxylapatitbeschichtung versehen ist.

9. Prothese (51) nach einem der Ansprüche 6-8, wobei der intramedulläre Stab (62) mindestens zwei Fixierungslöcher (56, 57) zum Verankern des intramedullären Stabes (62) in der Markhöhle (86) des Os metacarpale II (85) mit Winkelstabilität umfasst, wobei die mindestens zwei Fixierungslöcher (56, 57) in rechten Winkeln zur Längsachse des intramedullären Stabes (62) angeordnet sind und wobei die jeweiligen Achsen der Fixierungslöcher (56, 57) in einem Winkel größer als 5° zueinander, vorzugsweise in einem Winkel zwischen 45° und 135°, vorzugsweise in einem Winkel von 90° angeordnet sind.

10. Prothese (1; 31; 51) nach einem der vorstehenden Ansprüche, wobei das zweite Element (4; 34; 54) auch ein drittes Befestigungselement umfasst, das zur Befestigung an dem Os trapezoideum der Hand (12; 82) konfiguriert ist.

11. Prothese (1; 31; 51) nach einem der vorstehenden Ansprüche, wobei das erste Element (2; 52) mit dem zweiten Element (4; 34; 54) durch ein Kugelgelenk gekoppelt ist.

12. Prothese (1; 31; 51) nach Anspruch 11, wobei das Kugelgelenk einen ersten Teil umfasst, wobei der erste Teil einen mindestens teilweise becherförmigen Hohlraum umfasst und wobei der erste Teil auf dem ersten Element (2; 52) angeordnet ist, und wobei das Kugelgelenk einen zweiten Teil umfasst, wobei der zweite Teil eine mindestens teilweise kugelförmige Kugel (11; 41) umfasst, wobei der zweite Teil auf dem zweiten Element (4; 34; 54) angeordnet ist und wobei der kugelförmige Teil der Kugel (11; 41) drehbar in dem Hohlraum angeordnet ist.

13. Kit, umfassend eine Prothese (1; 31) nach einem der Ansprüche 4 oder 5 und mindestens ein Befestigungsmittel (16), wie beispielsweise einen mindestens teilweise mit Hydroxyapatit beschichteten Zapfen (16), wobei das mindestens eine Befestigungselement (16) länglich ist und auf oder nahe einem ersten äußeren Ende mit zweiten Verriegelungsmitteln zum Verankern des mindestens einen Befestigungselements (16) mit Winkelstabilität in mindestens einem des mindestens einen Befestigungslochs (6, 7; 37) bereitgestellt ist, das mit ersten Verriegelungsmitteln versehen ist, und an einem zweiten äußeren Ende zur Befestigung der Platte (5) an Os metacarpale II (15) mittels einer formschlüssigen Verbindung konfiguriert ist.

## Revendications

1. Prothèse (1 ; 31 ; 51) pour coupler de manière mobile l'os métacarpien I (14 ; 84) d'une main (12 ; 82) à un second os de la main (12 ; 82), laquelle prothèse (1 ; 31 ; 51) comprend un premier (2 ; 52) et un second élément (4 ; 34 ; 54), dans laquelle le premier élément (2 ; 52) comprend un premier élément de fixation (3 ; 53), lequel premier élément de fixation (3 ; 53) est configuré pour être fixé à l'os métacarpien I (14 ; 84), et le second élément (4 ; 34 ; 54) comprend un deuxième élément de fixation (5 ; 35 ; 55), lequel deuxième élément de fixation (5 ; 35 ; 55) est configuré pour être fixé au second os de la main (12 ; 82), lesquels premier (2 ; 52) et second éléments (4 ; 34 ; 54) peuvent se déplacer l'un par rapport à l'autre et dans laquelle le second os de la main (12 ; 82) est l'os métacarpien Il (15 ; 85) de la main (12 ; 82), **caractérisée en ce que** le deuxième élément de fixation (5 ; 35 ; 55) est configuré pour être fixé au côté radial de l'os métacarpien Il (15 ; 85).

2. Prothèse (1 ; 31) selon la revendication 1, dans laquelle le deuxième élément de fixation (4 ; 34) comprend une plaque (5 ; 35), laquelle plaque (5 ; 35) correspond sur un premier côté à la forme incurvée du côté externe radial de l'os métacarpien II (15) de la main (12).

3. Prothèse (1 ; 31) selon la revendication 2, dans laquelle la plaque (5 ; 35) comprend au moins un trou de fixation (6, 7 ; 37) pour recevoir un moyen de fixation, tel que, par exemple, une vis, une goupille ou une cheville, dans laquelle le au moins un trou de fixation (6, 7 ; 37) est continu et s'étend jusqu'au premier côté.

4. Prothèse (1 ; 31) selon la revendication 3, dans laquelle au moins un du au moins un trou de fixation (6, 7 ; 37) est muni d'un premier moyen de verrouillage, tel qu'un filetage interne ou une structure en relief, afin d'ancrer un moyen de fixation sur la plaque (5 ; 35) avec une stabilité angulaire par rapport à la plaque (5 ; 35).

5. Prothèse (1 ; 31) selon l'une quelconque des revendications 2-4, dans laquelle la plaque (5 ; 35) comprend au moins une broche de fixation (16 ; 42), laquelle au moins une broche de fixation (16 ; 42) s'étend depuis le premier côté et est configurée pour fixer la plaque (5 ; 35) à l'os métacarpien II (15).

6. Prothèse (51) selon la revendication 1, dans laquelle le deuxième élément de fixation (55) comprend une tige intramédullaire (62), laquelle tige intramédullaire (62) est allongée et est configurée pour une insertion intramédullaire, par le biais de la surface articulaire de l'os métacarpien Il (85) et du trapèze, dans la cavité médullaire (86) de l'os métacarpien II (85).

7. Prothèse (51) selon la revendication 6, dans laquelle la tige intramédullaire (62) est incurvée, dans laquelle la courbure correspond à la courbure de la cavité médullaire (86) de l'os métacarpien Il (85).

8. Prothèse (51) selon l'une quelconque des revendications 6 ou 7, dans laquelle la tige intramédullaire (62) est pourvue au moins partiellement d'un revêtement d'hydroxyapatite.

9. Prothèse (51) selon l'une quelconque des revendications 6-8, dans laquelle la tige intramédullaire (62) comprend au moins deux trous de fixation (56, 57) pour ancrer la tige intramédullaire (62) dans la cavité médullaire (86) de l'os métacarpien II (85) avec une stabilité angulaire, dans laquelle les au moins deux trous de fixation (56, 57) sont agencés à angle droit par rapport à l'axe longitudinal de la tige intramédullaire (62) et dans laquelle les axes respectifs des trous de fixation (56, 57) sont agencés selon un angle supérieur à 5° les uns par rapport aux autres, de préférence selon un angle compris entre 45° et 135°, de préférence selon un angle de 90°.

10. Prothèse (1 ; 31 ; 51) selon l'une quelconque des revendications précédentes, dans laquelle le second élément (4 ; 34 ; 54) comprend également un troisième élément de fixation configuré pour être fixé à l'os trapèze de la main (12 ; 82).

11. Prothèse (1 ; 31 ; 51) selon l'une quelconque des revendications précédentes, dans laquelle le premier élément (2 ; 52) est couplé au second élément (4 ; 34 ; 54) par une articulation sphérique.

12. Prothèse (1 ; 31 ; 51) selon la revendication 11, dans laquelle l'articulation sphérique comprend une première partie, laquelle première partie comprend une cavité au moins partiellement cupuliforme et laquelle première partie est agencée sur le premier élément (2 ; 52), et dans laquelle l'articulation sphérique comprend une seconde partie, laquelle seconde partie comprend une bille au moins partiellement sphérique (11 ; 41), laquelle seconde partie est agencée sur le second élément (4 ; 34 ; 54) et dans laquelle la partie sphérique de la bille (11 ; 41) est agencée de manière rotative dans la cavité.

13. Kit comprenant une prothèse (1 ; 31) selon l'une quelconque des revendications 4 ou 5 et au moins un moyen de fixation (16), tel qu'une cheville (16) au moins partiellement revêtue d'hydroxyapatite, dans lequel le au moins un moyen de fixation (16) est allongé et est pourvu sur une première extrémité externe, ou à proximité de celle-ci, d'un second moyen de verrouillage afin d'ancrer le au moins un moyen de fixation (16) avec une stabilité angulaire dans au moins un du au moins un trou de fixation (6, 7 ; 37) muni d'un premier moyen de verrouillage, et est configuré à une seconde extrémité externe pour la fixation de la plaque (5) à l'os métacarpien II (15) au moyen d'une connexion par ajustement de forme.
